Europäisches Patentamt

(19) European Patent Office

Office européen des brevets

(11) Numéro de publication : **0 033 292**
**B1**

(12) **FASCICULE DE BREVET EUROPÉEN**

(45) Date de publication du fascicule du brevet :
25.07.84

(51) Int. Cl.³ : **A 61 M 31/00, A 61 J 15/00**

(21) Numéro de dépôt : **81450001.3**

(22) Date de dépôt : **23.01.81**

(54) **Dispositif d'alimentation artificielle.**

(30) Priorité : **25.01.80 FR 8001936**

(43) Date de publication de la demande :
**05.08.81 Bulletin 81/31**

(45) Mention de la délivrance du brevet :
**25.07.84 Bulletin 84/30**

(84) Etats contractants désignés :
**BE CH DE GB IT LI NL SE**

(56) Documents cités :
**FR-A- 2 177 607**
**FR-A- 2 267 802**
**FR-A- 2 278 952**

(73) Titulaire : **DUBERNARD HOSPITAL S.A.**
**Quai de Bacalan 22**
**F-33000 Bordeaux (FR)**

(72) Inventeur : **Deckmyn, Jean-Claude**
**32, Résidence Pontet Lamartine**
**F-33600 Pessac (FR)**

(74) Mandataire : **Thébault, Jean-Louis**
**Cabinet Jean-Louis Thébault 50, Cours de Verdun**
**F-33000 Bordeaux (FR)**

Jouve, 18, rue St-Denis, 75001 Paris, France

## Description

La présente invention concerne les installations d'alimentation artificielle et plus particulièrement d'alimentation par voie entérale.

Ce type d'alimentation est utilisé lorsqu'un patient ne peut pas se nourrir pour des raisons physiopathologiques ou psychologiques alors que sa fonction digestive n'est pas entièrement perturbée, la substance nutritive étant introduite dans le système digestif à divers niveaux selon les cas par voie œsophagienne, gastrique ou entérale par exemple.

On connaît divers appareils ou les installations pour l'administration par voie entérale de telles substances nutritives ou alimentaires.

Ils sont chargés d'administrer des quantités ou rations prédéterminées en débit continu réglable, soit en une seule prise, soit en plusieurs prises échelonnées dans le temps.

Ces appareils comprennent généralement un récipient contenant la substance alimentaire, des moyens pour agiter cette dernière afin de lui conserver une bonne homogénéité et des moyens de propulsion, habituellement une pompe du type péristaltique, pour envoyer par l'intermédiaire d'une tubulure la substance alimentaire dudit récipient dans la sonde de nutrition du patient.

Les appareils connus comportent des récipients constitués par des flacons, bols ou fioles généralement en pyrex, recevant des substances alimentaires de viscosité variable et munis d'un système d'agitation à vitesse réglable constitué soit par un agitateur à pales mues mécaniquement soit par un barreau aimanté reposant sur le fond du récipient et entraîné par un champ magnétique tournant (FR-A-2 278 952).

Certains de ces appareils comportent un système de réchauffage ou de refroidissement constitué par un cristallisoir recevant le flacon d'aliment ainsi que de l'eau chaude ou de la glace pour délivrer l'aliment à la température désirée.

D'autres appareils comportent un système de réfrigération autonome de la substance alimentaire permettant ainsi une administration à faible débit, continue et autonome pendant 24 heures et plus.

Tous ces dispositifs présentent un certain nombre d'inconvénients.

Ceux qui sont munis d'un système de réchauffage ou refroidissement du type à bain Marie n'assurent pas avec précision et suffisamment longtemps le maintien à une température déterminée et ne sont pas d'un emploi simple et pratique.

Ceux, par contre, qui sont équipés d'un système de réfrigération autonome sont encombrants, lourds et coûteux.

On connaît aussi un dispositif d'alimentation artificielle ayant les caractéristiques énoncées dans le préambule de la revendication 1 (FR-A-2 177 607).

Tous, en revanche, présentent des inconvénients sur le plan de l'hygiène et de la sécurité du fait qu'ils utilisent comme récipient des aliments un flacon, bol ou fiole dont le contenu, au cours de l'utilisation du dispositif d'alimentation, n'est pas isolé de l'extérieur et risque d'être pollué ou contaminé. En effet, l'introduction des aliments dans le récipient sur les lieux de leur administration, la mise en place du bouchon d'obturation ou des organes de raccordement à la tubulure de transport ainsi qu'à l'introduction éventuelle du système d'agitation tel que l'agitateur à pales, entraînent des risques de pollution supplémentaires.

La présente invention a précisément pour but de supprimer ces divers inconvénients en proposant un dispositif d'alimentation entérale de conception simple, pratique, robuste et susceptible d'une très grande souplesse d'emploi dans des conditions d'hygiène et de sécurité optimales.

A cet effet l'invention a pour objet un dispositif d'alimentation artificielle, et plus particulièrement par voie entérale à l'aide d'une sonde alimentaire, du type comprenant une enceinte isothermique pourvue d'un moyen de compensation calorifique maintenant l'intérieur de l'enceinte à une température ou dans une plage de température déterminée pendant un temps déterminé, un récipient pour la substance alimentaire à administrer disposé, lors de l'utilisation du dispositif, à l'intérieur de l'enceinte isothermique, des moyens d'agitation de la substance alimentaire constitués, d'une part, par un barreau magnétique disposé à l'intérieur du récipient et, d'autre part, par un moteur d'entraînement en rotation du barreau, disposé à l'extérieur du récipient, un moyen de distribution de la substance alimentaire relié par tubulure audit récipient et à la sonde alimentaire, des moyens de support et de positionnement de l'enceinte, et des moyens de contrôle des divers paramètres du processus d'alimentation, ledit dispositif étant caractérisé en ce que ledit récipient à substance alimentaire est constitué par une poche souple en matière synthétique ou naturelle enfermée dans une boîte amovible constituant ladite enceinte isothermique et en ce que le moteur est disposé à l'extérieur de cette boîte.

Suivant un mode de réalisation préféré, la boîte isothermique est en matériau isothermique, de volume et de poids réduits, comportant deux demi-coquilles et au moins un orifice de passage de la tubulure reliant la poche souple au moyen de distribution des aliments, la boîte ayant une résistance suffisante pour servir d'emballage pour l'expédition et le stockage des poches souples.

Avantageusement, ledit moyen de compensation calorifique est constitué, notamment dans le cas de la boîte ci-dessus, par une substance dans la boîte de manière isolée et étanche, cette substance préalablement portée à une température déterminée étant introduite dans la boîte en même temps que la poche souple avec sa substance alimentaire et son barreau magnétique au moment de l'utilisation du dispositif d'alimentation artificielle.

Un tel dispositif est remarquable par sa simplicité, sa souplesse d'utilisation, son faible coût et par les excellentes conditions d'hygiène de son emploi.

En effet, les poches souples sont stérilisées ainsi que leur barreau magnétique avant le remplissage avec la substance alimentaire. Ce remplissage s'effectue en dehors des lieux d'utilisation du dispositif d'alimentation artificielle, dans de bonnes conditions d'hygiène, en sorte que sur les lieux d'utilisation l'intérieur des poches souples n'est pas mis directement en contact avec l'air ambiant au moment de la mise en route et du démarrage de l'installation d'alimentation. En outre, les poches souples ne servent qu'une fois, ce qui améliore encore les conditions d'hygiène.

La distribution de la substance alimentaire peut se faire soit par gravité en prévoyant un moyen de réglage du débit du type à étranglement sur la tubulure de raccordement de la poche souple à la sonde alimentaire. Elle peut se faire également, de préférence, par l'intermédiaire d'une pompe, par exemple de type péristaltique, l'enceinte isothermique avec sa poche souple, le moteur d'entraînement en rotation du barreau magnétique logé dans cette poche et la pompe avec ses organes de commande et de réglage étant avantageusement groupés et disposés sur un piètement support mobile.

D'autres caractéristiques et avantages ressortiront de la description qui va suivre d'un mode de réalisation du dispositif selon l'invention, description donnée à titre d'exemple uniquement et en regard des dessins annexés sur lesquels :

Figure 1 représente une vue en coupe schématique verticale d'une boîte isothermique selon l'invention à l'intérieur de laquelle sont disposés une poche souple remplie d'aliments et un bloc d'une substance à forte chaleur massique.

Figure 2 représente en perspective une demi-coquille d'une boîte isothermique selon l'invention,

Figure 3 représente une vue de dessus d'une poche souple utilisable dans le dispositif selon l'invention, la poche étant à plat et pourvue de son barreau magnétique,

Figure 4 illustre schématiquement un mode de réalisation d'un barreau magnétique ;

Figure 5 représente une vue en perspective d'un piètement support mobile sur lequel sont groupés les organes essentiels du dispositif d'alimentation artificielle,

Figure 6 représente une vue schématique latérale en coupe du dispositif de la Fig. 5, et

Figure 7 illustre la réalisation du moyen de compensation calorifique à l'aide d'un système de réfrigération à effet Peltier.

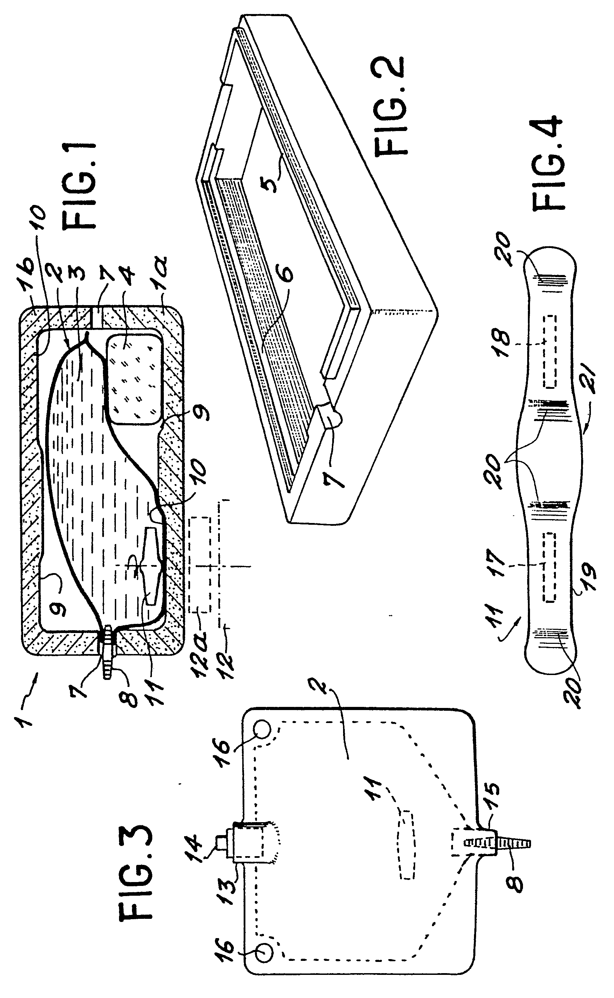

La figure 1 représente schématiquement une boîte isothermique 1 dans laquelle sont disposées une poche souple 2 remplie d'une substance alimentaire 3 et une masse, bloc ou quantité d'une substance 4 à forte chaleur massique, conformément à l'invention.

Le but de cette boîte est de maintenir, pendant un laps de temps déterminé, le contenu de la poche 2 dans une plage de température déterminée, ce contenu ayant été préalablement à sa mise en place porté à la température désirée, ladite substance 4 à forte chaleur massique jouant le rôle de volant thermique et étant, bien entendu, portée elle aussi à une température appropriée avant sa mise en place à l'intérieur de la boîte 1 simultanément avec la poche souple 2 remplie de sa substance alimentaire.

La boîte 1 a la forme d'une boîte d'allure générale parallélépipédique réalisée par exemple sous forme de deux demi-coquilles symétriques s'emboîtant l'une dans l'autre. La figure 2 représente l'une de ces coquilles.

Le matériau constitutif de la boîte est isothermique et léger, par exemple du polystyrène expansé ou un matériau analogue et, de préférence, ignifugé.

La boîte doit être légère, de volume réduit mais suffisamment résistante pour servir avantageusement d'emballage pour l'expédition et le stockage des poches souples 2 vides avant emploi, car il est préférable de jeter après usage les poches 2, par souci d'hygiène.

La réalisation de la boîte isothermique sous forme de deux demi-coquilles symétriques s'emboîtant est intéressante économiquement car elle ne nécessite qu'un seul type de moule. Ce type de boîte est bien connu et ne sera pas décrit en détail.

La demi-coquille représentée sur la figure 2 comporte sur la moitié de sa tranche périphérique une nervure 5 trouvant son complément dans l'autre moitié de sa périphérie sous forme d'un évidement 6 de dimensions et emplacement appropriés. En outre, afin de permettre la liaison de la poche souple 2 avec l'extérieur, la demi-coquille comporte dans la partie médiane de l'un de ses côtés latéraux, au niveau de la ligne de jonction avec l'autre demi-coquille, un évidement ou passage 7.

Bien entendu, comme la boîte sera obtenue par emboîtage de deux demi-coquilles identique, elle comportera un orifice à chaque extrémité. Lors de l'utilisation de la boîte, un seul des orifices sera utilisé, l'autre étant alors obturé, à l'aide d'une bande autocollante par exemple pour limiter le plus possible les échanges thermiques entre l'intérieur et l'extérieur de la boîte.

La boîte isothermique est, en cours d'utilisation du dispositif d'alimentation artificielle, disposée à plat comme représenté sur la figure 1 avec sa poche souple 2 remplie d'aliments placée à plat sur le fond de la demi-coquille inférieure 1a et tournée de manière que son embout 8 de raccordement se loge dans l'un des évidements 7 et fasse saillie vers l'extérieur, prêt à être connecté à la tubulure d'instillation des aliments.

La partie avant de la poche souple 2 repose sur le fond de la demi-coquille 1a dans une cuvette 10 cependant que la partie arrière repose sur le bloc 4 de substance à forte chaleur massique.

Généralement, ladite substance 4 aura pour but, afin de conserver de bonnes conditions de stérilité à l'intérieur de la boîte, de maintenir la température aux alentours de + 4 °C ± 2 °C et ce, pendant au moins 24 heures.

A cet effet, ladite substance est avantageusement du glycol conservé dans une poche de matière plastique et porté à une température inférieure à celle à maintenir dans l'enceinte, par exemple − 10 °C avant son introduction dans la boîte isothermique. Toute autre substance ayant des propriétés analogues peut être, bien entendu, utilisée pourvu qu'elle soit isolée physiquement et ne se répande pas à l'intérieur de la boîte. Ce pourrait être par exemple un bloc de glace sous poche.

Le positionnement respectif de la poche souple 2 et du bloc 4 de compensation calorifique est important, aussi il est intéressant de prévoir dans la paroi même de chaque demi-coquille 1a, 1b, un creux 9 réalisé au moulage et servant de repérage de l'emplacement du bloc 4 de glycol par exemple. Cet emplacement 9 est situé à l'opposé de l'évidement 7 de chaque demi-coquille.

Il est important, en effet, de placer la poche à aliment 2 dans ladite cuvette 10 au voisinage immédiat de l'orifice de sortie, et dans laquelle peut se mouvoir librement un barreau magnétique 11.

La substance alimentaire 3 est constituée de nutriments de compositions diverses et de viscosité plus ou moins grande. Elle doit être homogénéisée de manière continue. C'est le rôle du barreau magnétique 11 noyé dans la masse de substance 3 et entraîné en rotation par un moteur électrique extérieur à la boîte et schématisé en 12.

Pour que l'homogénéisation soit régulière d'un bout à l'autre de la prise alimentaire, il faut que le brassage s'effectue au voisinage de l'orifice de sortie de la poche souple 2 et qu'il s'opère même lorsque celle-ci est en fin de vidage et s'est presque complètement collabée.

La cuvette 10 assure un tel fonctionnement optimal de l'agitateur magnétique lequel ne s'arrête que lors du collabage de la poche 2.

Le moteur électrique 12 génère à la manière connue un champ magnétique tournant en regard du barreau 11. A cet effet, le moteur 12 entraîne par exemple un aimant symbolisé en 12a. Le positionnement du moteur 12 par rapport à la boîte 1 est donc également important car le barreau 11 doit pouvoir librement tourner, en dépit des forces de frottement et de l'écrasement progressif de la poche souple 2, le plus longtemps possible jusqu'à ce que la poche soit pratiquement vidée. Afin de faciliter ce vidage, la boîte isothermique 1 sera avantageusement inclinée de manière que la partie où se trouve le bloc 4 soit à un niveau légèrement plus élevé.

En outre, le dispositif 12 d'entraînement en rotation du barreau aimanté 11 doit être aussi près que possible de ce dernier et sera donc disposé contre la demi-coquille inférieure 1a ou à proximité immédiate de celle-ci, dans la demi-partie avant (côté de sortie de l'embout de raccordement 8), de manière à être sensiblement en regard du barreau 11 qui, par gravité, tombe au fond de la cuvette 10.

La poche souple contenant les nutriments est en matière plastique par exemple suivant plusieurs types connus. L'un est représenté sur la figure 3.

Cette figure représente une poche d'alimentation entérale, vide, de contour rectangulaire, comportant une tubulure 13 d'introduction des nutriments obturés par un bouchon 14, et une tubulure 15 d'évacuation de diamètre plus petit et dans lequel est emmanché un embout de raccordement 8.

Des trous 16 sont prévus à la partie supérieure de la poche en vue de suspendre cette dernière à un portique lors du remplissage.

Afin de faciliter l'écoulement des nutriments, la partie intérieure de la poche au voisinage de l'embout 8 va en se rétrécissant en direction de ce dernier. C'est dans cette partie que se loge le barreau aimanté 11 lorsque la poche, une fois remplie, est posée à plat dans la demi-coquille inférieure 1a suivant des repères de positionnement prévus.

D'autres types de poches peuvent également convenir. C'est ainsi que la poche peut ne comporter qu'une seule tubulure servant au remplissage et à l'évacuation et obturée par un bouchon muni d'un passage de tubulure avec un embout de raccordement du genre de l'embout de raccordement de la tubulure d'administration des nutriments qui doit être positionné dans le passage 7 prévu dans la boîte isothermique 2.

La figure 4 illustre un mode de réalisation d'un barreau magnétique 11 particulièrement adapté au dispositif de l'invention.

Ce barreau 11 est constitué de deux petits barreaux cylindriques 17 et 18, de ferrite par exemple, enfilés dans un tube 19 en matière plastique alimentaire et isolés dans deux compartiments par pinçage et soudage du tube 19. On a symbolisé en 20 les zones de pinçage-soudage.

De plus, les plans de pinçage des deux zones 20 centrales sont décalés l'un par rapport à l'autre de 90° en sorte que les deux bandes de soudure centrales 20 sont orthogonales. Ceci donne une partie renflée 21 au centre du barreau 11 sur tout son pourtour, ce qui facilite sa rotation sur le fond de la poche en limitant les frottements avec ce fond.

La longueur du barreau 11 est par exemple de l'ordre de 8 cm.

L'embout de raccordement 8 de la poche à nutriments est relié en cours d'utilisation du dispositif à une tubulure d'administration faisant office de réchauffer pour disposer d'un nutriment à 19-20 °C à l'entrée de la sonde alimentaire.

L'instillation du nutriment peut se faire par simple gravité, le débit étant réglé par exemple par une pince ou un dispositif d'étranglement de la tubulure d'administration du type goutte à goutte.

De préférence, l'administration se fait à l'aide d'une pompe, par exemple du type péristaltique, interposée dans le circuit d'une tubulure d'administration appropriée.

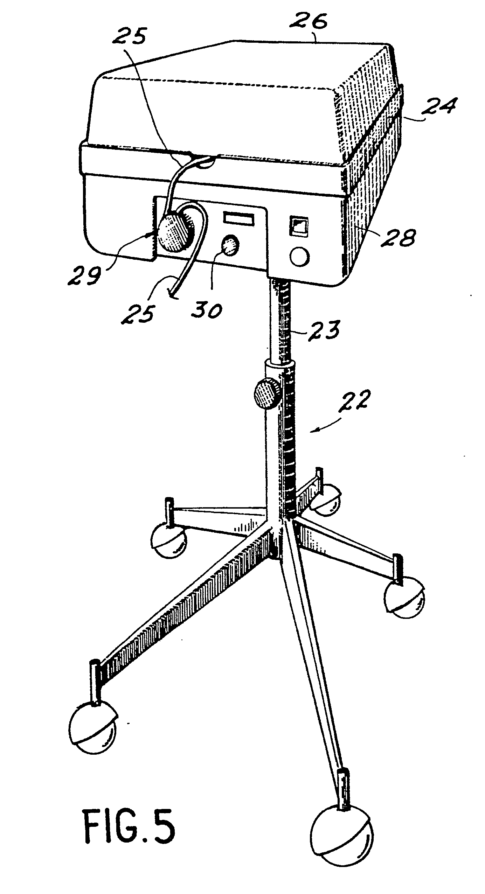

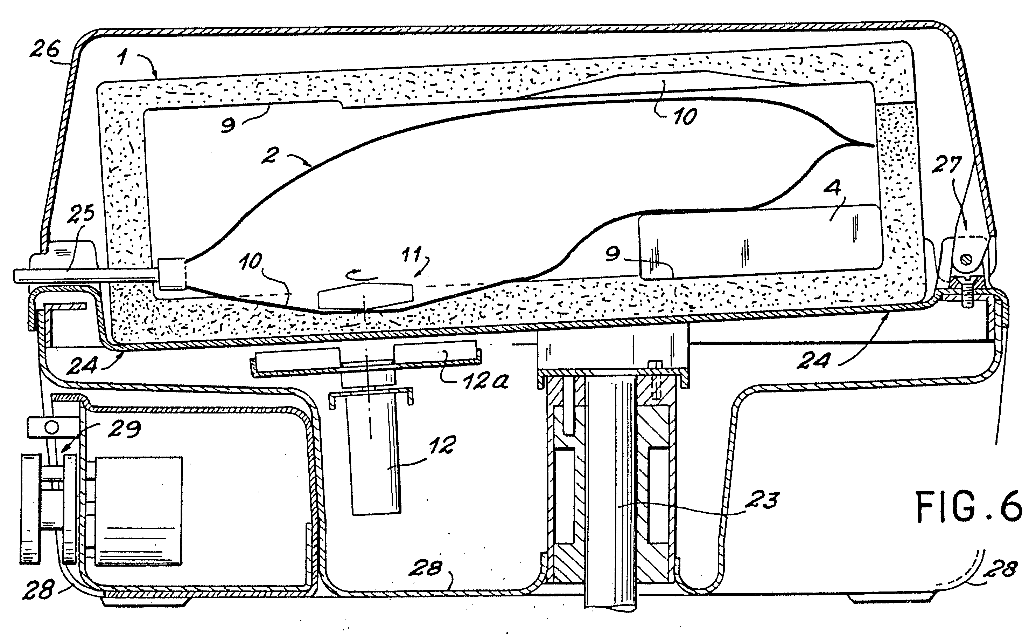

Les figures 5 et 6 illustrent un mode de réalisation d'un appareillage permettant de grouper les organes essentiels du dispositif de l'invention et de faciliter son utilisation.

A cet effet, l'appareillage comprend un piètement 22 à pied 23 réglable en hauteur, monté sur roulettes.

Le pied 23 supporte un bac 24 légèrement incliné vers l'avant (côté sortie du nutriment) et recevant une boîte isothermique 1 selon l'invention, dans laquelle ont été mis en place une poche de nutriment avec son barreau magnétique et un bloc de glycol par exemple.

Le bac 24 est étanche, isolant et amovible, afin de permettre son nettoyage.

La boîte 1 est masquée par un capot 26 basculant autour d'une articulation 27 prévue à l'arrière du bac 24.

En dessous du bac 24 sont montés sur un carter-support 28 fixé au bac 24 les organes de commande, de contrôle et de réglage de l'administration du nutriment. Ces organes comprennent une pompe 29, par exemple péristaltique, interposée dans la tubulure d'administration 25. Il s'agit par exemple d'une pompe à vitesse variable permettant soit une alimentation continue, soit une alimentation discontinue.

A côté de la pompe 29 et de sa commande sont disposés le moteur 12 d'entraînement du barreau magnétique et ses organes de commande.

Le moteur 12 est un moteur électrique à vitesse variable et est placé sous le bac 24 en dessous de la partie médiane avant de la boîte 1 comme on l'a expliqué plus haut. Ses organes de contrôle 30 sont disposés sur la face avant de la console constituée par le carter-support 28.

Par souci de clarté, on n'a pas représenté sur les figures 5 et 6 les connexions d'alimentation électrique.

Le mode d'utilisation du dispositif d'alimentation artificielle est particulièrement destiné aux milieux hospitaliers bien qu'il puisse être également utilisé à domicile.

La substance alimentaire ou nutriment est introduite dans une poche d'alimentation entérale préalablement munie d'un barreau magnétique, l'ensemble poche-barreau étant stérilisé avant remplissage.

Avantageusement, les poches vides neuves sont livrées aux utilisateurs munies de leur barreau, stérilisées et conditionnées par paquets expédiés directement à l'intérieur de la boîte isothermique qui sert d'emballage et de stockage.

Par mesure d'hygiène, le remplissage s'effectue avantageusement, notamment en milieu hospitalier, en dehors des lieux d'administration. La poche une fois remplie est munie de son bouchon et mise au froid.

Son contenu ne sera plus par la suite en contact avec le milieu extérieur, sauf pour le branchement de la tubulure d'administration sur l'embout de raccordement de la poche. Lorsque le moment de la prise alimentaire est arrivé, la poche est sortie du froid et mise dans une boîte isothermique 1 en même temps qu'un bloc de glycol par exemple, puis la boîte est amenée sur les lieux d'administration où se trouve l'appareillage des figures 5 et 6 par exemple.

Le couvercle 26 est soulevé, la boîte 1 mise en place, la demi-coquille supérieure provisoirement retirée pour permettre le raccordement de l'embout de la poche 2 à la tubulure d'administration et l'administration du nutriment peut commencer après avoir refermé la boîte 1 et rabattu le couvercle 26.

Elle peut s'opérer de manière continue ou discontinue et suivant un débit réglable. De même, le degré d'agitation du nutriment dans sa poche est réglé en fonction de sa nature et de sa viscosité.

Avec le dispositif de l'invention, on n'effectue sur les lieux d'administration des aliments qu'une seule opération très simple de mise en place de la boîte isothermique, sans aucun contact du contenu de la poche avec le milieu extérieur, sauf pour la connexion de la tubulure d'administration, ce qui diminue considérablement les risques de pollution et contamination du nutriment.

Malgré la température de l'ordre de + 4 °C à l'intérieur de la boîte isothermique, le nutriment a le temps de se réchauffer au cours de son transit dans la tubulure d'administration.

On pourrait, bien entendu, si cela est souhaitable, introduire dans la boîte une substance chargée de maintenir la température à un niveau différent, par exemple supérieure à la température ambiante. Il suffit d'introduire la substance à forte chaleur massique à la température nécessaire, le volant thermique constitué par cette substance jouant son rôle aussi bien dans un sens que dans l'autre.

Il est à noter que la compensation calorifique à l'intérieur de l'enceinte isothermique, en particulier le maintien à des températures de quelques degrés, pourrait être réalisé à l'aide d'un système de réfrigération électrique à effet Peltier.

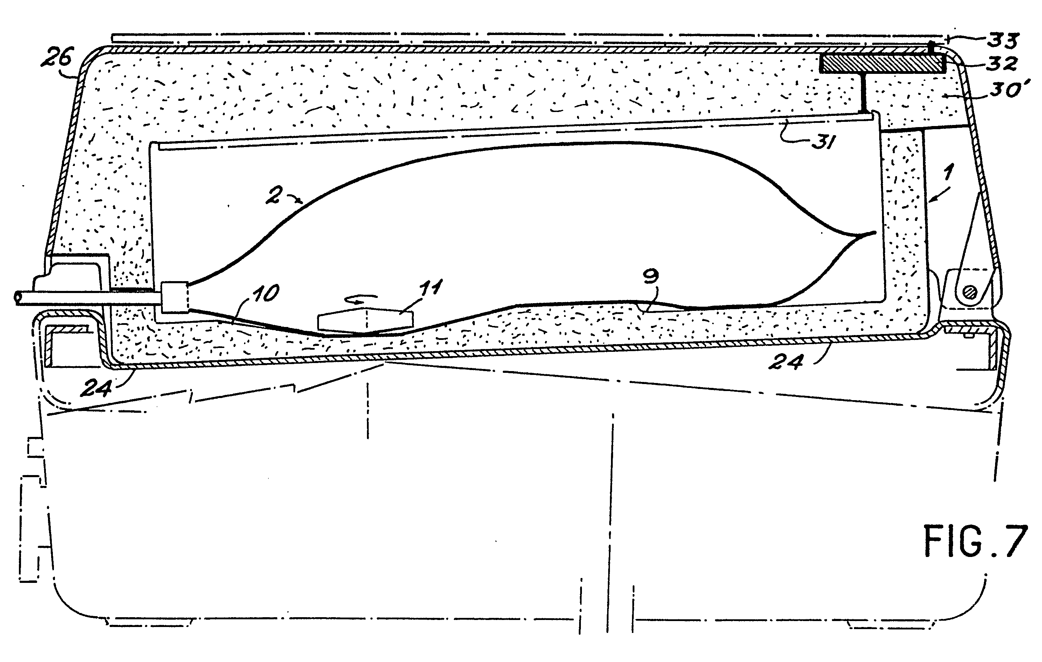

On a représenté sur la figure 7 une variante de réalisation mettant en œuvre un dispositif de réfrigération à effet Peltier.

Dans cette variante, le couvercle 26 de l'appareillage des figures 5 et 6 est appelé à se substituer à la demi-coquille supérieure de la boîte isothermique 1.

A cet effet, le couvercle 26 comporte intérieurement un garnissage 30' en matériau isolant thermique conformé de manière, lorsque le couvercle 26 est rabattu, à isoler la poche 2 exactement comme le faisait la demi-coquille supérieure de la boîte 1 avant son enlèvement.

Sur la face interne du garnissage 30' est disposé en 31 l'échangeur froid d'un dispositif à effet Peltier comprenant une cellule à effet Peltier 32 noyée dans le matériau de garnissage et un échangeur chaud 33 fixé à l'extérieur du couvercle 26. On n'a pas représenté sur la figure 7 les connexions électriques de la cellule 32.

## Revendications

1. Dispositif d'alimentation artificielle, et plus particulièrement par voie entérale à l'aide d'une sonde alimentaire, du type comprenant une enceinte isothermique (1) pourvue d'un moyen de compensation calorifique (4) maintenant l'intérieur de l'enceinte (1) à une température ou dans une plage de température déterminée pendant un temps déterminé, un récipient (2) pour la substance alimentaire (3) à administrer disposé, lors de l'utilisation du dispositif, à l'intérieur de l'enceinte isothermique (1), des moyens d'agitation de la substance alimentaire (3) constitués, d'une part, par un barreau magnétique (11) disposé à l'intérieur du récipient (2) et, d'autre part, par un moteur (12) d'entraînement en rotation du barreau (11), disposé à l'extérieur du récipient (2), un moyen (29) de distribution de la substance alimentaire (3) relié par tubulure (25) audit récipient (2) et à la sonde alimentaire, des moyens (22, 24) de support et de positionnement de l'enceinte (1), et des moyens (30) de contrôle des divers paramètres du processus d'alimentation, ledit dispositif étant caractérisé en ce que ledit récipient à substance alimentaire est constitué en ce que ledit récipient à substance alimentaire est constitué par une poche souple (2) en matière synthétique ou naturelle enfermée dans une boîte amovible (1) constituant ladite enceinte isothermique et en ce que le moteur (12) est disposé à l'extérieur de cette boîte (1).

2. Dispositif suivant la revendication 1 caractérisé en ce que la boîte isothermique (1) est en matériau isothermique de volume et de poids réduits, comportant deux demi-coquilles (1a, 1b) et au moins un orifice (7) de passage de la tubulure (25) reliant la poche souple (2) au moyen (29) de distribution des aliments, la boîte ayant une résistance suffisante pour servir d'emballage pour l'expédition et le stockage des poches souples.

3. Dispositif suivant la revendication 1 ou 2 caractérisé en ce que ledit moyen de compensation calorifique (4) est constitué par une substance à forte chaleur massique, en quantité suffisante et disposée dans la boîte isothermique (1) de manière isolée et étanche, cette substance préalablement portée à une température déterminée étant introduite dans la boîte (1) en même temps que la poche souple (2) avec sa substance alimentaire (3) et son barreau magnétique (11) au moment de l'utilisation du dispositif d'alimentation artificielle.

4. Dispositif suivant la revendication 3 caractérisé en ce que la boîte isothermique (1) est constituée de deux demi-coquilles symétriques emboîtables (1a, 1b), chaque demi-coquille étant pourvue, dans son fond, d'un creux (9) ou emplacement matérialisé, disposé à l'opposé d'un orifice (7) de passage de la tubulure (25) de connexion à la poche d'aliment (2) et destiné à servir de repère pour la mise en place de la substance (4) de compensation calorifique.

5. Dispositif suivant l'une des revendications 2 à 4 caractérisé en ce que la poche à aliment (2) comporte un embout de raccordement (8) traversant ledit orifice (7) de passage, de la boîte isothermique (1) et connecté à la tubulure (25) d'administration des aliments, ledit embout étant emmanché sur la poche ou solidaire d'un bouchon de fermeture de la poche.

6. Dispositif suivant l'une des revendications 1 à 5 caractérisé en ce que ledit barreau magnétique (11) est constitué d'un ou plusieurs barreaux (17, 18) retenus prisonniers dans un manchon tubulaire (19) en matière plastique pincé et soudé aux deux extrémités et dans sa partie centrale, de manière à former dans cette dernière partie un renflement (21) servant de pivot facilitant la rotation du barreau magnétique.

7. Dispositif suivant l'une des revendications 1 à 6 caractérisé en ce que ledit moyen de distribution de la substance alimentaire est constitué par une pince ou dispositif d'étranglement de la tubulure d'administration (25) disposé sur celle-ci et réglant le débit de l'écoulement, celui-ci s'effectuant par gravité.

8. Dispositif suivant l'une des revendications 1 à 6 caractérisé en ce que ledit moyen de distribution de la substance alimentaire est constitué par une pompe (29), de préférence du type péristaltique, à débit réglable.

9. Dispositif suivant la revendication 4 caractérisé en ce que chaque demi-coquille (1a, 1b) de la boîte isothermique (1) comporte, dans la partie de son fond située entre l'orifice de passage (7) et le creux (9) de positionnement de la substance (4) de compensation calorifique, une cuvette (10) destinée à faciliter le positionnement du barreau magnétique (11).

10. Dispositif suivant la revendication 1 ou 2 caractérisé en ce que le moyen de compensation calorifique (4) est constitué par un système de réfrigération à effet Peltier comprenant une cellule à effet Peltier (32), un échangeur froid (31) disposé dans la boîte isothermique (1) et un échangeur chaud (33) disposé à l'extérieur de ladite boîte.

11. Dispositif suivant la revendication 8 caractérisé en ce qu'il comporte un piètement-support (22, 23) mobile sur lequel est monté un plateau (24) légèrement incliné d'un côté constituant l'avant du dispositif, le plateau recevant la boîte isothermique (1) placée de manière que l'orifice de sortie de la poche à aliment (2) soit dirigé vers ledit avant, le dispositif agitateur (11, 12) et la pompe (29) étant disposés en dessous du plateau (24) ainsi que leurs organes de contrôle (30).

12. Dispositif suivant les revendications 10 et 11 caractérisé en ce qu'il comporte un couvercle amovible (26) muni sur sa face intérieure d'un garnissage (30') en matériau isolant thermique conformé de manière à se substituer à la demi-coquille supérieure (1b) d'une boîte isothermique et comportant une cellule à effet Peltier (32) reliée à un échangeur froid (31) disposé sur la face interne dudit garnissage et à un échangeur chaud (33) disposé sur la face externe du couvercle.

**Claims**

1. An artificial feeding device, and more particularly a device for feeding artificially enterally by means of a feeding probe of the type comprising an isothermic enclosure (1) provided with a calorific compensation means (4) maintaining the inside of the enclosure (1) at a determined temperature or within a determined temperature range for a determined period of time, a container (2) for the food substance (3) to be administered which is positioned inside the isothermic enclosure (1) when the device is being used, means for agitating the food substance (3) which, on the one hand, consist of a magnetic bar (11) positioned inside the container (2) and on the other hand, a driving motor (12) for rotating the bar (11), which motor is positioned outside the container (2), a means (29) for distributing the food substance (3) which is connected by a tube (25) to the abovementioned container (2) and to the feeding probe, means (22, 24) for supporting and positioning the enclosure (1) and means (30) for controlling the various parameters of the geeding process, said device being characterised in that the above-mentioned contained for the food substance consists of a flexible bag (2) made of synthetic or natural material and is contained in a removable box (1) forming the above-mentioned isothermic enclosure, and the motor (12) is positioned outside this box (1).

2. A device according to Claim 1, characterised in that the isothermic box (1) is made of isothermic material having a reduced volume and weight, comprising two shell-haves 1a, 1b and at least one orifice (7) for the passage of the tube (25) connecting the flexible bag (2) to the means (29) for distributing the food, the box being strong enough to be used as a packing for the dispatch and storage of the flexible bags.

3. A device according to Claim 1 or 2, characterised in that the above-mentioned calorific compensation means (4) consists of a substance having a considerable heat as regards mass, in a sufficient quantity, and is positionned in the isothermic box (1) in an insulated and tight manner, this substance having been previously brought to a determined temperature and being introduced into the box (1) at the same time as the flexible bag (2) with its food substance (3) and its magnetic bar (11) just before the artificial feeding device is used.

4. A device according to Claim 3, characterised in that the isothermic box (1) consists of two symmetrical shell-halves, 1a, 1b which may be fitted together, each shell-half being provided in its bottom with a hollow (9) or a materialised position located opposite an orifice (7) for the passage of the tube (25) for connecting the food bag (2) and to serve as a reference mark for the positionning of the calorific compensation substance (4).

5. A device according to one of Claims 2-4, characterised in that the food bag (2) has a connecting nozzle (8) passing through the above-mentioned orifice (7) for the passage of the isothermic box (1) and connected to the tube (25) for administering the food, this nozzle being attached to the bag or being integral with a stopper for closing the bag.

6. A device according to one of Claims 1-5, characterised in that the magnetic bar (11) consists of one or more bars (17, 18) which are firmly held in a tubular sleeve (19) made of plastics material which is pinched and soldered at its two ends and in its centre part in order to form in this last part an enlargement (21) acting as a pivot facilitating the rotation of the magnetic bar.

7. A device according to one of Claims 1-6, characterised in that the means for distributing the food substance consists of pinchers or a device for constricting the administration tube (25), which device is positioned on this tube (25) and which regulates the flow rate which takes place under the effects of gravity.

8. A device according to one of Claims 1-6, characterised in that the means for distributing the food substance consists of a pump (29), preferably of the peristaltic type, having an adjustable flow rate.

9. A device according to Claim 4, characterised in that each shell-half, 1a, 1b of the isothermic box (1) comprises in that part of its bottom located between the passage orifice (7) and the hollow (9) for positioning the calorific compensation substance (4) a basin (10) to facilitate the positioning of the magnetic bar (11).

10. A device according to Claim 1 or 2, characterised in that the calorific compensation means (4) consists of a Peltier effect refrigeration system comprising a Peltier effect cell (32), a cold exchanger (31) positioned in the isothermic box (1) and a hot exchanger (33) positioned outside the box (1).

11. A device according to Claim 8, characterised in that it comprises a mobile supporting mounting (22, 23) on which a plate (24) is mounted which is slightly inclined on one side and which forms the front of the device, the plate receiving the isothermic box (1) which is positioned so that the outlet orifice of the food bag (2) is directed towards the front of the device, the agitating device (11, 12) and the pump (29) being positioned below the plate (24) as are their control members (30).

12. A device according to Claims 10 and 11, characterised in that it comprises a removable cover (26) which is provided on its inside surface with a lining (30') made of shaped, thermally insulating material in order to take the place of the upper shell-half 1b of an isothermic box, and comprising a Peltier effect cell (32) connected to a cold exchanger (31) which is positionned on the internal surface of said lining and to a hot exchanger (33) positioned on the external surface of the cover.

**Ansprüche**

1. Vorrichtung zur künstlichen Ernährung, insbesondere über den Darmweg mittels einer Ernährungssonde, eines Typs umfassend einen isothermischen Raum (1), der mit einem Mittel zur kalorischen Kompensation (4) versehen ist, das das Innere des Raums (1) während einer vorbestimmten Zeit auf einer vorbestimmten Temperatur oder in einem vorbestimmten Temperaturbereich hält, einen Behälter (2) für die zu verabreichende Nahrungsmittelsubstanz (3), der während des Gebrauchs der Vorrichtung im Inneren des isothermischen Raums (1) angeordnet ist, Mittel zum Rühren der Nahrungsmittelsubstanz (3), die einerseits durch einen magnetischen Stab (11), der im Inneren des Behälters (2) angeordnet ist, und andererseits durch einen Motor (12) zum Versetzen des Stabs (11) in Rotation, der außerhalb des Behälters (2) angeordnet ist, gebildet werden, ein Mittel (29) zum Austeilen der Nahrungsmittelsubstanz (3), das über Leitungen (25) mit dem Behälter (2) und der Ernährungssonde verbunden ist, Mittel (22, 24) zum Tragen und Positionieren des Raums (1) und Mittel (30) zum Steuern von verschiedenen Parametern des Ernährungsvorgangs, wobei die Vorrichtung dadurch gekennzeichnet ist, daß der Behälter für die Nahrungsmittelsubstanz aus einem weichen Sack (2) aus synthetischem oder natürlichem Material gebildet wird, der in einem abnehmbaren Gehäuse (1), das den isothermischen Raum bildet, eingeschlossen ist, und daß der Motor (12) ausserhalb des Gehäuses (1) angeordnet ist.

2. Vorrichtung nach Anspruch 1, dadurch gekennzeichnet, daß das isothermische Gehäuse (1) aus einem isothermischen Material mit verringertem Volumen und Gewicht besteht und zwei Halbschalen (1a, 1b) und wenigstens eine Öffnung (7) für den Durchgang der Leitung (25), die den weichen Sack (2) mit dem Mittel (29) zum Austeilen von Nahrungsmitteln verbindet, aufweist, wobei das Gehäuse eine genügende Festigkeit besitzt, um als Verpackung für den Transport und die Lagerung von weichen Säcken zu dienen.

3. Vorrichtung nach Anspruch 1 oder 2, dadurch gekennzeichnet, daß das Mittel zur kalorischen Kompensation (4) aus einer Substanz mit hoher spezifischer Wärmekapazität gebildet ist, die in ausreichender Menge und in dem isothermischen Gehäuse (1) isoliert und dicht angeordnet ist, wobei diese Substanz, die vorher auf eine vorbestimmte Temperatur gebracht wurde, gleichzeitig mit dem weichen Sack (2) mit dessen Nahrungsmittelsubstanz und dessen magnetischem Stab (11) im Moment des Gebrauchs der Vorrichtung zur künstlichen Ernährung eingebracht wird.

4. Vorrichtung nach Anspruch 3, dadurch gekennzeichnet, daß das isothermische Gehäuse (1) aus zwei symmetrischen ineinandersteckbaren Halbschalen (1a, 1n) besteht, wobei jede Halbschale auf ihrem Boden mit einer Vertiefung (9) oder einem materialisierten Standort versehen ist, die bzw. der gegenüberliegend von der Öffnung (7) für den Durchgang der Verbindungsleitung (25) für den Nahrungsmittelsack (2) ange

ordnet und dazu bestimmt ist, als die Stelle für das Anordnen der Substanz (4) zur kalorischen Kompensation zu dienen.

5. Vorrichtung nach einem der Ansprüche 2 bis 4, dadurch gekennzeichnet, daß der Nahrungsmittelsack (2) ein Verbindungsstück (8) umfaßt, daß sich durch die Durchtrittsöffnung (7) des isothermischen Gehäuses (1) erstreckt und mit der Leitung (25) zur Verabreichung von Nahrungsmitteln verbunden ist, wobei das Verbindungsstück an den Sack angestielt oder fest mit einem Verschlußstopfen für den Sack verbunden ist.

6. Vorrichtung nach einem der Ansprüche 1 bis 5, dadurch gekennzeichnet, daß der magnetische Stab (11) aus mehreren Stäben gebildet ist, die in einer schlauchförmigen Hülle (19) aus Plastikmaterial eingeschlossen gehalten sind, die an beiden Enden und in ihrem mittleren Teil eingedrückt und verschweißt ist, so daß in diesem letzteren Teil ein Wulst (21) gebildet wird, der als Drehachse dient, um die Rotation des magnetischen Stabes zu erleichtern.

7. Vorrichtung nach einem der Ansprüche 1 bis 6, dadurch gekennzeichnet, daß das Mittel zum Austeilen der Nahrungsmittelsubstanz durch eine Klemme oder eine Vorrichtung zum Einschnüren der Versorgungsleitung (25) gebildet wird, die auf dieser angeordnet ist und die Ausflußmenge regelt, wobei der Ausfluß durch Schwerkraft erfolgt.

8. Vorrichtung nach einem der Ansprüche 1 bis 6, dadurch gekennzeichnet, daß das Mittel zum Austeilen der Nahrungsmittelsubstanz durch eine Pumpe (29), vorzugsweise eine peristaltische, mit regulierbarer Leistung gebildet wird.

9. Vorrichtung nach Anspruch 4, dadurch gekennzeichnet, daß jede Halbschale (1a, 1b) des isothermischen Gehäuses (1) in ihrem Bodenbereich zwischen der Durchtrittsöffnung (7) und der Vertiefung (9) zum Positionieren der Substanz (4) zur kalorischen Kompensation eine Mulde (10) zum Erleichtern der Anordnung des magnetischen Stabes (11) aufweist.

10. Vorrichtung nach Anspruch 1 oder 2, dadurch gekennzeichnet, daß das Mittel zur kalorischen Kompensation (4) aus einem Kühlsystem auf der Basis des Peltier-Effektes gebildet wird, das eine Peltier-Zelle (32), einen Kältetauscher (31), der in dem isothermischen Gehäuse (1) angeordnet ist, und einen Wärmetauscher (33) umfaßt, der außerhalb dieses Gehäuses angeordnet ist.

11. Vorrichtung nach Anspruch 8, dadurch gekennzeichnet, daß sie ein bewegliches Trägergestell (22, 23) umfaßt, auf dem eine Platte (24) montiert ist, die leicht zu der Seite, die die Vorderseite der Vorrichtung bildet, geneigt ist, wobei die Platte, die das isothermische Gehäuse (1), das derart angeordnet ist, daß die Austrittsöffnung des Nahrungsmittelsacks (2) zur Vordersete gerichtet ist, die Rühreinrichtung (11, 12) und die Pumpe (29) aufnimmt, die unter der Platte (24) ebenso wie ihre Steuerorgane (30) angeordnet ist.

12. Vorrichtung nach den Ansprüchen 10 und 11, dadurch gekennzeichnet, daß sie einen abnehmbaren Deckel (26) umfaßt, der an seiner Innenseite mit einer Auskleidung (30') aus thermisch isolierendem Material versehen ist, die derart gestaltet ist, daß sie die obere Halbschale (1b) des isothermischen Gehäuses ersetzt und eine Peltier-Zelle (32) enthält, die mit einem Kältetauscher (31), der an der Innenseite der Auskleidung angeordnet ist, und einem Wärmetauscher (33), der an der Außenseite des Deckels angeordnet ist, verbunden ist.

FIG.1

FIG.2

FIG.3

FIG.4

FIG.5

FIG.6

0 0 33 292

26

33

32

30'

31

2

1

10

11

9

24

24

0 033 292

FIG. 7